# FASCICULE DE BREVET EUROPEEN

(11) **EP 2 870 192 B1**
(45) Date de publication et mention de la délivrance du brevet: **06.12.2017**
(21) Numéro de dépôt: 13744715.7
(22) Date de dépôt: 08.07.2013
(51) Int. Cl.: C08G 69/36, A61K 8/88, A61Q 19/00, C08J 3/12, C08G 69/26, C08L 77/00

(54) **POUDRE DE POLYAMIDE HYDRODISPERSIBLE**
WASSERDISPERGIERBARES POLYAMIDPULVER
WATER-DISPERSIBLE POLYAMIDE POWDER

(30) Priorité: 06.07.2012 FR 1256546
(43) Date de publication de la demande: 13.05.2015
(73) Titulaire: Arkema France, 92700 Colombes (FR)
(72) Inventeur: PINEAU, Quentin, F-27000 Evreux (FR)
(86) Numéro de dépôt international: PCT/FR2013/051624
(87) Numéro de publication internationale: WO 2014/006353

(56) Documents cités:
- EP-A1- 0 062 138
- WO-A1-2012/001299

## Description

### Domaine de l'invention

La présente invention a pour objet une poudre de polyamide hydrodispersible, et en particulier la fabrication de dispersions aqueuses stables de fines particules de polyamide.
Par « dispersion stable », on entend au sens de l'invention une dispersion qui ne flocule pas, dont les particules de polyamide ne s'agrègent pas, même après 24 mois à température de 20°C. Les particules de poudre de la dispersion stable peuvent toutefois sédimenter, mais sont capables de se redisperser spontanément par simple agitation à température ambiante.
Par « hydrodispersible », on entend une poudre de polyamide qui, introduite dans l'eau à 20°C, à une concentration massique comprise dans la gamme de 0,5 à 90%, permet l'obtention d'une solution macroscopiquement homogène.
Par « fines particules», on entend des particules de diamètre médian en volume (ci-après D50) inférieur ou égal à 50 µm.
Au sens de l'invention, le « D50 » correspond à la taille moyenne en volume, c'est à dire la valeur de la taille de particule qui divise la population de particules examinée exactement en deux. Le D50 est mesuré selon la norme ISO 9276 - parties 1 à 6 : « Représentation de données obtenues par analyse granulométrique ».

### Technique antérieure

Les dispersions aqueuses de polyamide sont largement utilisées dans les encres, les films de revêtement, le traitement des textiles, l'entoilage textile, le traitement du papier, les peintures, les lubrifiants, les adhésifs hot melt (HMA). Ces dispersions aqueuses sont difficiles à obtenir à partir des polyamides disponibles commercialement, destinés à une utilisation industrielle, qui se présentent généralement sous forme de poudres ou de granulés. Les poudres de polyamide existantes ne sont pas, telles quelles, dispersibles directement dans un milieu aqueux, et ne peuvent donc pas former de dispersion aqueuse homogène stable.

En effet, les procédés connus de fabrication de dispersions aqueuses de polyamide nécessitent généralement de nombreuses étapes, sont techniquement très complexes, et coûteux en énergie. Dans ces procédés, le polyamide est fondu puis dispersé dans un milieu aqueux au moyen de solvant(s) organique(s) et/ou de dispersant(s) selon différentes méthodes par exemple décrites dans les documents de brevet WO9747686 ou WO9844062. En particulier, les polyamides de haut poids moléculaire nécessitent de grandes quantités de solvant organique, et forment des solutions extrêmement visqueuses difficiles à disperser en milieux aqueux.

Il n'est pas non plus possible de préparer une dispersion de polyamide en milieu aqueux par un procédé direct de polymérisation en émulsion à cause du procédé de fabrication du polyamide, typiquement par polycondensation ou par ouverture de cycle. Les procédés actuels se basent donc soit sur une reprécipitation, soit sur une post-émulsification. La reprécipitation consiste à dissoudre le polyamide dans un solvant organique, à reprécipiter le polyamide sous forme de particules puis à remplacer le solvant organique par un milieu aqueux. Mais la taille des particules ainsi obtenues est trop grande (D50 supérieur à 50 µm) pour donner une dispersion stable ce qui fait obstacle à l'utilisation de telles dispersions dans les encres et les peintures. En effet, dans ces dispersions, les particules on tendance à s'agréger, voire à floculer, dans le milieu aqueux de sorte que la dispersion aqueuse n'est pas stable. Enfin, le fait d'inclure l'étape supplémentaire de remplacement du solvant organique par un milieu aqueux complique le procédé classique de reprécipitation pour fabriquer de la poudre de polyamide. Dans la post-émulsification, le polyamide est d'abord dissous dans un solvant organique de façon à préparer une solution, puis la solution de polyamide est mélangée à un émulsifiant dans un milieu aqueux, et émulsifiée au moyen d'un fort cisaillement pour former une dispersion aqueuse de polyamide. Mais le polyamide n'est que peu soluble dans les solvants organiques de sorte que le procédé de post-émulsification n'est pas viable économiquement, sa productivité étant trop faible. En outre, le procédé de post-émulsification utilise de grandes quantités de solvants organiques, facteurs de pollution environnementale. Enfin, une dispersion aqueuse de polyamide obtenue par post-émulsification contient inévitablement des résidus de solvants organiques et d'émulsifiants.

Les techniques existantes ne permettent pas non plus d'obtenir des dispersions aqueuses stables à forte concentration en polyamide, notamment si la concentration en polyamide dépasse les 50% en poids dans le milieu aqueux.

Pour pouvoir être utilisées dans le domaine des encres et des peintures, les particules de polyamide dans la dispersion aqueuse doivent être très fines, de D50 inférieur ou égal à 50 µm. Des dispersions aqueuses de polyamides couramment employées dans les encres, les liants et les adhésifs, sont celles qui contiennent de fines particules de copolyamides à base de dimères d'acides gras. Leur procédé d'obtention, décrit notamment dans le document de brevet Henkel (US5804682), nécessite de solubiliser le copolyamide (ci-après COPA) à base de dimère d'acide gras dans un alcool « léger » (type isobutanol) avant d'y ajouter de l'eau, un tensioactif, et des agents épaississants pour former une émulsion, puis d'évaporer l'alcool pour récupérer une dispersion aqueuse de COPA à base de dimère d'acide gras. Ces dispersions aqueuses de COPA à base de dimère d'acide gras doivent contenir un tensioactif à base de nonylphénol toxique et cancérigène pour pouvoir rester stables. Comparativement aux autres copolyamides, les copolyamides à base de dimères d'acides gras ont comme particularités d'être beaucoup moins cristallins, de faible résistance chimique (vis-à-vis des solvants), de faible module et sont très tackifiants ce qui ralentit la productivité des procédés qui utilisent ce genre de dispersions. Les COPA dimères d'acides gras sont solubles dans les alcools légers en C1 à C6 à une seule fonction alcool et de bas point d'ébullition (température d'ébullition inférieure à 120°C). En revanche, les COPA autres que dimères d'acides gras sont insolubles dans ce type d'alcools légers à une seule fonction alcool, tels que l'isobutanol. Pour la grande majorité des polyamides qui sont plus cristallins que ces COPA dimères d'acides gras, ce procédé notamment décrit dans US5804682 n'est donc pas envisageable.

Il existe donc un réel besoin de fournir des dispersions aqueuses utilisant tous types de polyamides, y-compris des polyamides autres que les copolyamides à base de dimères d'acides gras.

Par exemple, certains copolyamides sont connus comme adhésifs du type "hot melt adhesives" (ou HMA), c'est-à-dire qu'on les dépose à l'état fondu sur les surfaces à coller, l'adhésion étant ensuite obtenue par refroidissement, par le retour à l'état solide des copolyamides. Le point de fusion de ces COPA HMA est généralement compris dans la gamme de 80 à 190°C, de préférence de 100 à 130°C. La composition en monomères et le ratio en poids entre les monomères déterminent notamment les propriétés d'adhérence sur différents supports et la résistance chimique de ces COPA. Ces propriétés des COPA HMA sont déjà exploitées dans le textile, dans la fabrication de films, filaments, voiles ou grilles, de vernis, de peintures, d'encres et le revêtement de matériaux, notamment sous la forme de poudres fines de COPA. Toutefois, ces poudres ne sont pas hydrodispersibles. De plus, il s'avère très difficile par broyage atmosphérique, ou même par broyage cryogénique à une température inférieure à la Tg (température de transition vitreuse du polymère), généralement à -100°C, d'obtenir des poudres de COPA HMA de D50 inférieur à 60 µm. En effet, les procédés utilisés sont très coûteux et très peu productifs, entraînent beaucoup de pertes, nécessitent plusieurs recyclages et passages dans le broyeur, consomment beaucoup de temps et d'énergie, et nécessitent en plus de sélectionner la poudre par tamisage. Ceci est lié au fait que les HMA on tendance à devenir élastiques et à s'échauffer ce qui rend leur broyage particulièrement difficile.

La présente invention a donc pour but de fournir un procédé de fabrication de poudres de PA hydrodispersibles, et de dispersions aqueuses de polyamide, notamment de COPA, tel que les COPA HMA, prêtes à l'emploi, qui facilitent leur mise en oeuvre par les formulateurs, en étant directement utilisables (par simple incorporation) dans des formulations.

La présente invention a notamment pour but de fournir un procédé simple (comprenant le moins d'étapes possibles) de fabrication de telles poudres de polyamides prêtes à l'emploi, à partir d'un polyamide quelle que soit sa forme : granulé, poudre, liquide (fondu) ou solide, etc.

De manière surprenante, la Demanderesse a montré que l'ajout d'un acide de Brönsted à base de phosphore (ci-après abrégé « acide P »), tel que l'acide phosphorique, à un mélange de polyamide et d'eau, permettait dans certaines conditions spécifiques à l'invention, de fabriquer des dispersions aqueuses de polyamide stables et même d'obtenir une morphologie atypique de poudre fine de polyamide, ayant la particularité d'être hydrodispersible.

### Description détaillée de l'invention

Dans la présente description, on précise que lorsqu'il est fait référence à des intervalles, les expressions du type «allant de... à...» ou « comportant/comprenant de...à... » incluent les bornes de l'intervalle. A l'inverse, les expressions du type « compris entre... et... » excluent les bornes de l'intervalle.

La présente invention a donc pour objet une poudre à base de particules de polyamide dans laquelle :
- ledit polyamide comporte plus de 50% molaire d'extrémités amines sur le nombre total d'extrémités amines et acides du polyamide;
- lesdites particules comportent à leur surface des groupements amines primaires au moins partiellement neutralisés par un acide de Brönsted à base de phosphore (« acide P »), de préférence de l'acide phosphorique ;
- la D50 des particules est comprise dans la gamme de 100 nm à 50 µm, de préférence de 100 nm à 20 µm.

Par acide de Brönsted à base de phosphore, on entend tout acide comprenant du phosphore et susceptible de céder un proton c'est-à-dire un ion H+ en milieu aqueux. L'acide de Brönsted à base de phosphore comprend notamment au moins un des acides suivants : acide hypophosphoreux H3PO2, acide phosphoreux H3PO3, acide phosphorique H3PO4, acide perphosphorique H3PO5 et leurs dérivés tels que l'hypophosphite de sodium NaH2PO2, l'hypophosphite de potassium KH2PO2, et leurs mélanges. De préférence, l'acide de Brönsted à base de phosphore comprend de l'acide phosphorique, et plus préférablement est constitué d'acide phosphorique H3PO4.

Avantageusement, ladite poudre de polyamide selon l'invention comprend au moins un homopolyamide et/ou au moins un copolyamide (COPA).

Avantageusement, ladite poudre de polyamide comprend au moins un monomère choisi parmi les acides aminocarboxyliques, de préférence alpha,oméga-aminocarboxyliques, comprenant de 4 à 18 atomes de carbone, les couples diamine.diacide comprenant de 4 à 36 atomes de carbone, les lactames comprenant de 3 à 18 atomes de carbone, et leurs mélanges.

Selon un mode de réalisation préféré de l'invention, lesdites particules à base de polyamide comprennent au moins un polyamide et/ou au moins un copolyamide et/ou leurs mélanges.

Par polyamide (homopolyamide ou copolyamide) au sens de l'invention on entend les produits de condensation des lactames, des aminoacides et/ou des diacides avec les diamines et, en règle générale, tout polymère formé par des motifs ou monomères reliés entre eux par des groupes amides.

Le terme « monomère » dans la présente description des poudres de polyamides doit être pris au sens d' « unité répétitive ». Le cas où une unité répétitive du polyamide est constituée de l'association d'un diacide avec une diamine est particulier. On considère que c'est l'association d'une diamine et d'un diacide, c'est-à-dire le couple diamine.diacide (en quantité équimolaire), qui correspond au monomère. Ceci s'explique par le fait qu'individuellement, le diacide ou la diamine n'est qu'une unité structurale, qui ne suffit pas à elle seule à polymériser. Dans le cas où les particules de poudre selon l'invention comprenant au moins deux monomères différents, appelés «co-monomères», c'est à dire au moins un monomère et au moins un co-monomère (monomère différent du premier monomère), elles comprennent un copolymère tel qu'un copolyamide abrégé COPA.

Par copolyamide (abrégé COPA), on entend les produits de polymérisation d'au moins deux monomères différents choisis parmi :
- les monomères de type aminoacides ou acides aminocarboxyliques, et de préférence les acides alpha,oméga-aminocarboxyliques;
- les monomères de type lactames ayant de 3 à 18 atomes de carbone sur le cycle principal et pouvant être substitués ;
- les monomères de type « diamine.diacide » issus de la réaction entre une diamine aliphatique ayant de 4 et 36 atomes de carbone, de préférence de 4 à 18 atomes de carbone et un diacide carboxylique ayant de 4 et 36 atomes de carbone, de préférence de 4 à 18 atomes de carbone ; et
- leurs mélanges, avec des monomères à nombre de carbone différent dans le cas de mélanges entre un monomère de type aminoacide et un monomère de type lactame.

### Monomères de type aminoacides :

A titre d'exemples d'alpha,oméga-aminoacides, on peut citer ceux ayant de 4 à 18 atomes de carbone, tels que les acides aminocaproïque, 7-aminoheptanoïque, 11-aminoundécanoïque, N-heptyl-11-aminoundécanoïque et 12-aminododécanoïque.

### Monomères de type lactames :

A titre d'exemples de lactames, on peut citer ceux ayant de 3 à 18 atomes de carbone sur le cycle principal et pouvant être substitués. On peut citer par exemple le β,β-diméthylpropriolactame, le α,α-diméthylpropriolactame, l'amylolactame, le caprolactame aussi appelé lactame 6, le capryllactame aussi appelé lactame 8, l'oenantholactame et le lauryllactame aussi appelé lactame 12.

### Monomères de type « diamine.diacide » :

A titre d'exemples d'acide dicarboxylique, on peut citer les acides ayant de 4 et 36 atomes de carbone de carbone. On peut citer par exemple, l'acide adipique, l'acide sébacique, l'acide azélaïque, l'acide subérique, l'acide isophtalique, l'acide butanedioïque, l'acide 1,4 cyclohexyldicarboxylique, l'acide téréphtalique, le sel de sodium ou de lithium de l'acide sulphoisophtalique, les acides gras dimérisés (ces acides gras dimérisés ont une teneur en dimère d'au moins 98% et sont de préférence hydrogénés) et l'acide dodécanédioïque HOOC-(CH2)₁₀-COOH, et l'acide tétradécanedioïque.

On entend plus particulièrement par dimères d'acides gras ou acides gras dimérisés, le produit de la réaction de dimérisation d'acides gras (contenant généralement 18 atomes de carbone, souvent un mélange d'acide oléique et/ou linoléique). Il s'agit de préférence d'un mélange comprenant de 0 à 15% de monoacides en C18, de 60 à 99% de diacides en C36, et de 0,2 à 35% de triacides ou polyacides en C54 ou plus.
A titre d'exemple de diamine, on peut citer les diamines aliphatiques ayant de 4 à 36 atomes, de préférence de 4 à 18 atomes, pouvant être aryliques et/ou cycliques saturées. A titre d'exemples on peut citer l'hexaméthylènediamine, la pipérazine (abrégée « Pip »), l'aminoethylenepiperazine, la tetraméthylène diamine, l'octaméthylène diamine, la décaméthylène diamine, la dodécaméthylène diamine, le 1,5 diaminohexane, le 2,2,4-triméthyl-1,6-diamino-hexane, les polyols diamine, l'isophorone diamine (IPD), le méthyl pentaméthylènediamine (MPMD), la bis(aminocyclohéxyl) méthane (BACM), la bis(3-méthyl-4 aminocyclohéxyl) méthane (BMACM), la méthaxylyènediamine, et le bis-p aminocyclohexylméthane.

A titre d'exemples de monomères de type « diamine.diacide », on peut citer ceux résultant de la condensation de l'hexaméthylène diamine avec un diacide C6 à C36, notamment les monomères : 6.6, 6.10, 6.11, 6.12, 6.14, 6.18. On peut citer les monomères résultant de la condensation de la décanediamine avec un diacide C6 à C36, notamment les monomères : 10.10, 10.12, 10.14, 10.18.

De préférence, les poudres de polyamide de l'invention comprennent au moins un polyamide choisi parmi les polyamides et copolyamides comprenant au moins un des monomères suivants : 4.6, 4.T, 5.6, 5.9, 5.10, 5.12, 5.13, 5.14, 5.16, 5.18, 5.36, 6, 6.6, 6.9, 6.10, 6.12, 6.13, 6.14, 6.16, 6.18, 6.36, 6 .T, 9, 10.6, 10.9, 10.10, 10.12, 10.13, 10.14, 10.16, 10.18, 10.36, 10.T, 11, 12, 12.6, 12.9, 12.10, 12.12, 12.13, 12.14, 12.16, 12.18, 12.36, 12.T, et leurs mélanges; en particulier choisi parmi le PA 11, le PA 12, le PA 10.10, le PA 6, le PA 6.10, PA 10.12, PA 6.14 et/ou PA 6.6/6, le PA 6/12, le PA 11/10.10, et leurs mélanges.

A titre d'exemples de copolyamides formés à partir des différents types de monomères décrits ci-dessus, on peut citer les copolyamides résultant de la condensation d'au moins deux acides alpha,oméga-aminocarboxyliques ou de deux lactames ou d'un lactame et d'un acide alpha,oméga-aminocarboxylique. On peut encore citer les copolyamides résultant de la condensation d'au moins un acide alpha,oméga-aminocarboxylique (ou un lactame), au moins une diamine et au moins un acide dicarboxylique. On peut encore citer les copolyamides résultant de la condensation d'une diamine aliphatique avec un diacide carboxylique aliphatique et d'au moins un autre monomère choisi parmi les diamines aliphatiques différentes de la précédente et les diacides aliphatiques différents du précédent.

A titre d'exemples de copolyamides, on peut citer des copolymères de caprolactame et de lauryllactame (PA 6/12), des copolymères de caprolactame, d'hexaméthylène diamine et d'acide adipique (PA 6/6.6), des copolymères de caprolactame, de lauryllactame, d'hexaméthylène diamine et d'acide adipique (PA 6/12/6.6), des copolymères de caprolactame, d'hexaméthylène diamine et d'acide azélaïque, d'acide 11-aminoundécanoïque, et de lauryllactame, (PA 6/6.9/11/12), des copolymères de caprolactame, d'acide adipique et d'hexaméthylène diamine, d'acide

11-aminoundécanoïque, de lauryllactame (PA 6/6.6/11/12), des copolymères d'hexaméthylène diamine, d'acide azélaïque, et de lauryllactame (PA 6.9/12), des copolymères de caprolactame et d'acide 11-aminoundécanoïque (PA 6/11), des copolymères de lauryllactame et de capryllactame (PA 12/8), des copolymères de capryllactame et de caprolactame (PA 8/6), des copolymères de lauryllactame et de capryllactame (PA 12/8), des copolymères de lauryllactame et d'acide 11-aminoundécanoïque (PA 12/11).

Avantageusement, le COPA utilisé dans la composition selon l'invention est obtenu au moins partiellement à partir de matières premières bio-ressourcées.
Par matières premières d'origine renouvelable ou matières premières bio-ressourcées, on entend des matériaux qui comprennent du carbone bio-ressourcé ou carbone d'origine renouvelable. En effet, à la différence des matériaux issus de matières fossiles, les matériaux composés de matières premières renouvelables contiennent du ¹⁴C. La « teneur en carbone d'origine renouvelable » ou « teneur en carbone bio-ressourcé » est déterminée en application des normes ASTM D 6866 (ASTM D 6866-06) et ASTM D 7026 (ASTM D 7026-04). A titre d'exemple, les COPA à base de polyamide 11 proviennent au moins en partie de matières premières bio-ressourcées et présentent une teneur en carbone bio-ressourcé d'au moins 1%, ce qui correspond à un ratio isotopique de ¹²C/¹⁴C d'au moins 1,2 x 10⁻¹⁴. De préférence, les COPA selon l'invention comprennent au moins 50% en masse de carbone bio-ressourcé sur la masse totale de carbone, ce qui correspond à un ratio isotopique ¹²C/¹⁴C d'au moins 0,6.10⁻¹². Cette teneur est avantageusement plus élevée, notamment jusqu'à 100%, qui correspond à un ratio isotopique ¹²C/¹⁴C de 1,2 x 10⁻¹², dans le cas de COPAs issus en totalité de matières premières d'orgine renouvelable.

A titre d'exemples d'aminoacides d'origine renouvelable, on peut citer: l'acide 11-aminoundécanoïque produit à partir d'huile de ricin par exemple, l'acide 12-aminododécanoïque produits à partir d'huile de ricin par exemple, l'acide 10-aminodécanoïque produit à partir de l'acide décylénique obtenu par métathèse de l'acide oléïque par exemple, l'acide 9-aminononanoïque produit à partir de l'acide oléïque par exemple.

A titre d'exemples de diacides d'origine renouvelable, on peut citer, en fonction du nombre x de carbones de la molécule (Cx) :
- C4 : l'acide succinique à partir du glucose par exemple ;
- C6 : l'acide adipique à partir du glucose par exemple ;
- C7 : l'acide heptanedioïque à partir d'huile de ricin ;
- C9 : l'acide azélaïque à partir de l'acide oléïque (ozonolyse) par exemple ;
- C10 : l'acide sébacique à partir de l'huile de ricin par exemple ;
- C11 : l'acide undécanedioïque à partir d'huile de ricin ;
- C12: l'acide dodécanedioïque à partir de biofermentation de l'acide dodecanoïque = acide laurique (huile riche : huile de palmiste et noix de coco) par exemple ;
- C13 : acide brassylique à partir de l'acide erucique (ozonolyse) que l'on trouve dans le colza par exemple ;
- C14 : acide tetradécanedioïque par biofermentation de l'acide myristique (huile riche : huile de palmiste et noix de coco) par exemple ;
- C16 : acide hexadécanedioïque par biofermentation de l'acide palmitique (huile de palme principalement) par exemple ;
- C18 : acide octadécanedioïque obtenu par biofermentation de l'acide stéarique (un peu dans toutes les huiles végétales mais majoritaire dans les graisses animales) par exemple ;
- C20 : acide eicosanedioïque obtenu par biofermentation de l'acide arachidique (majoritaire dans l'huile de colza) par exemple ;
- C22 : acide docosanedioïque obtenu par métathèse de l'acide undécylénique (huile de ricin) par exemple
- C36 : dimère d'acide gras issu des sous-produits des résineux transfomés par les procédé Kraft.

A titre d'exemples de diamines d'origine renouvelable, on peut citer, en fonction du nombre x de carbones de la molécule (Cx) :
- C4 : butanediamine obtenu par amination de l'acide succinique ;
- C5 : pentaméthylene diamine (à partir de lysine) ;
et ainsi de suite pour les diamines obtenues par amination des diacides d'origine renouvelable vus précédemment.
Par copolyamide d'origine totalement renouvelable, on entend les copolyamides résultant de la polymérisation de divers monomères (renouvelables, non renouvelables ou mixtes) tels que ceux cités ci-dessus. C'est le cas par exemple du COPA 6.6/10.10 dans lequel le monomère « 6.6 » est d'origine non renouvelable tandis que le monomère « 10.10 » est d'origine renouvelable.
Par copolyamide d'origine totalement renouvelable entrant dans la composition selon l'invention, on entend les copolyamides résultant de la polymérisation de divers monomères, tels que ceux cités ci-dessus, comme par exemple les copolyamides suivants: PA 11/10.10, PA 11/10.36, PA 10.10/10.36, le copolyamide 11-aminoundécanoïque/N-heptyl-11-aminoundécanoïque, etc.
On utilise avantageusement un ou plusieurs des copolyamides suivants dans la composition ou le procédé de la présente invention :
- PA 6/6.6/12, dont les ratios massiques en monomères correspondants peuvent être (en pourcentage) : 40/20/40, 35/20/45, 45/35/20, 30/30/40, 22/18/60, 40/25/35 ;
- PA 6/6.6/11/12, dont les ratios massiques en monomères correspondants peuvent être par exemple (en pourcentage) : 30/15/10/45, 30/20/20/30, ou 15/25/25/35 ;
- PA 6/12 de ratio massique 70/30 ;
- PA 6.9/12 de ratio massique 30/70 ;
- PA Pip.9/Pip.12/11 de ratio massique 15/70/15 ;
- PA 6/IPD.6/12 de ratio massique 20/15/65 ;
- PA IPD.9/12 de ratio massique 20/80 ;
- PA6/MPMD.12/12 de ratio massique 27/33/33 ;
- PA 6/6.12/12 de ratio massique 30/30/40 ;
- PA 6/Pip.12/12 de ratio massique 30/20/50 ;
- PA 6/6.12/11/PEG.12 de ratio massique 25/21/25/30 ;
- PA 6.10/11/PEG.10 de ratio massique 14/14/42/30 ;
- PA 6/6.6/6.10/6.1 de ratio massique 40/10/40/10 ;
- PA 6.10/Pip.10/Pip.12 de ratio massique 20/40/40 ;
- PA 6/11/12 de ratio massique 10/36/54 ;
- PA Pip.12/12 de ratio massique 35/65 ;
- PA IPD.10/12 de ratio massique 80/20 ;
- PA Pip.10/12 de ratio massique 72/28 ;
- PA 6/11 de ratio massique 50/50 ;
- PA Pip.10/11/Pip.9 de ratio massique 65/30/5 ;
- PA 6/6.6/6.10 de ratio massique 35/30/35
A titre d'exemples de copolyamides, on peut notamment citer ceux commercialisés sous le nom Platamid® et Platamid® Rnew par ARKEMA, Vestamelt® par Evonik, et Griltex® par EMS.
De préférence le polyamide utilisé dans la présente invention comprend au moins un copolyamide choisi parmi : PA 6/6.6/12, PA 6/6.6/11/12, PA 6/12, PA 6.9/12, PA Pip.9/Pip.12/11, PA 6/IPD.6/12, PA IPD.9/12, PA6/MPMD.12/12, PA 6/6.12/12, PA 6/Pip.12/12, PA 6/6.6/6.10/6.I, PA 6.10/Pip.10/Pip.12, PA 6/11/12, PA Pip.12/12, PA IPD.10/12, PA Pip.10/12, PA 6/11, PA Pip.10/11/Pip.9, PA 6/6.6/6.10, et en particulier ceux dont les ratios massiques sont définis plus haut, et des mélanges de ces copolyamides.
Les extrémités ou fins de chaîne de ces polyamides selon l'invention sont majoritairement des extrémités amines. Il est possible d'adapter les extrémités des polyamides par l'utilisation de composés polyfonctionnels et de limiteurs de chaîne, généralement des composés mono fonctionnels, au cours de leur synthèse.
Par composé polyfonctionnel, on entend un composé ayant plus de 2 fonctions acide(s) et/ou amine(s). On peut citer à titre d'exemples, la diéthylène triamine (DETA); une polyéthertriamine, telle que la Jeffamine T403 ; la Bis(Hexamethylene)Triamine ; l'EDTA ; l'acide mellitique.
Par composé limiteur de chaîne, on entend un composé monofonctionnel ayant 1 fonction réactive acide ou amine, tel que l'acide acétique, l'acide laurique, l'acide stéarique, l'acide undécylénique, la laurylamine.
On utilise de préférence les composés monoamines et/ou diamines, pour obtenir des (co)polyamides conformes à l'invention qui possèdent des extrémités de chaîne majoritairement amine. En particulier, lors de la synthèse de ces (co)polyamides utilisés dans la présente invention, les réactions de condensation se déroulent en excès de fonctions amine.

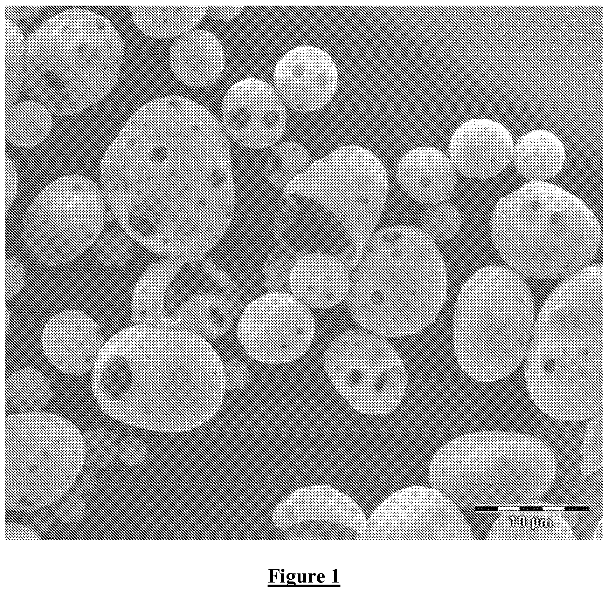

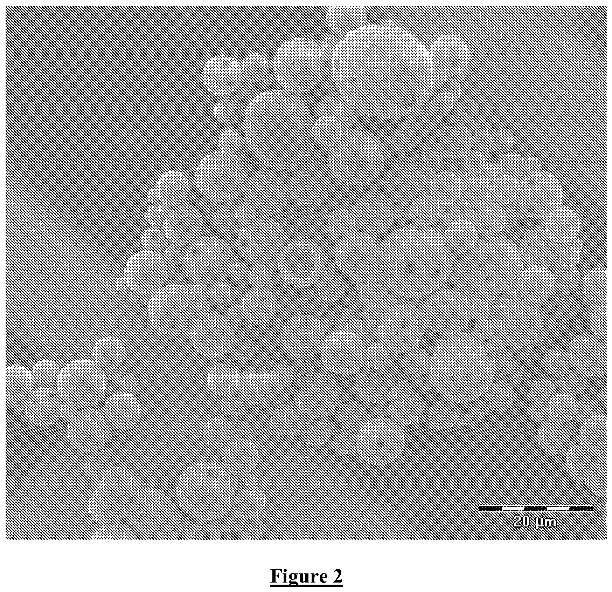

Avantageusement, la poudre de polyamide de l'invention comprend des particules qui sont à la fois :
- de forme sphéroïdale,
- dont la surface ou paroi de la particule est percée ou poreuse,
- et de préférence creuses, c'est-à-dire que le coeur (ou partie centrale) des particules est vide,
comme celles représentées sur les photos de la figure 1 et de la figure 2, observées au microscope électronique à balayage, sur l'appareil MEB Philips XL30FEG.

De préférence, la poudre selon l'invention contient au mois 5%, de préférence au mois 15%, de préférence au moins 30%, de préférence au moins 50%, voire au moins 70%, ou mieux au moins 90% en poids de particules creuses, sur le poids total de particules.

Avantageusement, le coeur des particules de l'invention contient au moins un produit cosmétique, pharmaceutique ou de parfumerie. Cette morphologie creuse particulière des poudres selon l'invention leur permet de s'imprégner de ces produits non pas seulement en surface mais en profondeur, au coeur même de la particule et de contenir une quantité plus importante de produits que les poudres de l'art antérieur poreuses en surface uniquement.

Dans la présente description, la granulométrie des poudres est mesurée par diffraction laser, sur appareil Coulter® LS230, selon la norme ISO 9276.

Concernant les caractéristiques chimiques des poudres, le pourcentage d'extrémités amines est mesuré par dosage potentiométrique (acido-basique), de préférence par l'acide perchlorique.

On vérifie que les groupements amines primaires à la surface des particules d'une poudre de polyamide sont au moins partiellement neutralisés, notamment par un acide de Brönsted à base de phosphore tel que l'acide phosphorique, si la poudre polyamide se disperse dans l'eau à température ambiante pour former un liquide laiteux par simple agitation. Dans le cas contraire, notamment si la poudre ne forme pas avec l'eau un liquide d'aspect laiteux sous agitation, et qu'elle tombe au fond du récipient malgré l'agitation, les groupements amines primaires à la surface des poudres ne sont pas au moins partiellement neutralisés.

Les propriétés physico-chimiques de la poudre selon l'invention font qu'elle est dispersible dans un milieu aqueux de pH neutre ou acide, tel que de l'eau, ledit milieu ayant un pH compris dans la gamme de 1 à 7, de préférence de 4 à 6,5, pour former une composition homogène ou dispersion stable.

Par milieu aqueux de pH neutre ou acide, on entend au sens de l'invention, tout milieu liquide comprenant au moins 50% d'eau, de préférence au moins 70% d'eau, de préférence au moins 80% d'eau, de préférence au moins 90% d'eau, de préférence 100% d'eau, et de pH compris dans la gamme de 1 à 7, de préférence de 4 à 6,5. L'eau peut être de l'eau adoucie, de l'eau déionisée, de l'eau distillée, de l'eau déminéralisée et/ou de l'eau stérilisée, selon son degré de purification, de l'eau thermale, etc.

Le milieu aqueux peut en outre comprendre des alcools miscibles avec l'eau, de préférence dont le nombre de carbones de la chaîne carbonée ne dépasse pas 6, tels que l'éthanol ou l'isopropanol. Des solutions alcooliques obtenues par simple mélange de ces alcools avec l'eau sont également utilisables dans le milieu aqueux; de même que les glycols, comme l'éthylène glycol, propylène glycol ; les polyols, comme le glycérol ou glycérine, le sorbitol, le sirop de sorbitol.

Les polyoxyéthylènes glycols (PEG) peuvent également être utilisés comme solvants dans ledit milieu aqueux. Les polymères carboxyvinyliques (carbomères ou Carbopol), les polymères cyanoacryliques ; les composés glucidiques, tels que polysaccharides ou polyosides extraits d'algues (alginates, carragénates), du bois (cellulose et ses dérivés), de la sève des arbres (gomme arabique, gomme adragante), de graines ou de pépins (pectine, gommes de guar, de caroube, amidon), de feuilles (gel d'aloès) ; les glycoprotéines ou protéoglycanes ; les esters et éthers glucidiques peuvent également entrer dans la composition du milieu aqueux, notamment comme épaississants ou gélifiants du milieu aqueux.

Avantageusement, des conservateurs, émulsionnants hydrophiles, colorants, humectants, gélifiants, actifs hydrophiles et tout autre agent cosmétique hydrophile, peuvent entrer dans la composition dudit milieu aqueux.
La présente invention a notamment pour objet une composition fluide homogène comprenant :
- de 0,5 à 90% en poids de poudre telle que définie précédemment,
- de 10 à 99,5% en poids d'un milieu aqueux de pH neutre ou acide. Avantageusement, la composition comprend :
- de 0,5 à 60% en poids de poudre selon l'invention, et
- de 40 à 99,5% en poids d'un milieu aqueux de pH neutre ou acide, de préférence de pH compris dans la gamme de 1 à 7, de préférence de 4 à 6,5. De préférence, le milieu aqueux est de l'eau et ladite composition forme une dispersion aqueuse stable sans tensioactif.

La dispersion aqueuse selon l'invention a la forme d'une dispersion macroscopiquement homogène, ayant un aspect laiteux plus ou moins épais en fonction de la concentration de polyamide dans la dispersion, mais toujours d'aspect homogène. L'expression « composition fluide homogène » signifie que la poudre et le milieu aqueux ne peuvent pas être distingués à l'oeil nu après agitation. Si la composition est homogène on ne peut alors observer qu'un liquide.

Ces propriétés des dispersions aqueuses de poudre selon l'invention sont vérifiées, même à des concentrations élevées en polyamide, par exemple de 60 à 70% en poids de polyamide sur le poids total de la dispersion, et ceci même en l'absence de tensioactif.

Les poudres et dispersions aqueuses de la présente invention sont particulièrement adaptées à certaines applications, notamment textiles et composites, pour lesquelles on préfère travailler à pH acide, ce qui garantit une meilleure adhérence du polyamide sur le textile.

Les dispersions aqueuses de la présente invention sont aussi adaptées à la formulation de cosmétiques qui nécessitent un pH neutre ou acide, adapté à la peau, de préférence compris dans la gamme de pH allant de 5,5 à 6,5.

Grâce à la poudre de polyamide selon l'invention, le formulateur n'a plus besoin d'adapter au préalable la forme des polyamides vendus dans le commerce sous forme de poudre ou de granulés pour pouvoir les incorporer dans de l'eau.

De préférence, ledit milieu aqueux de pH neutre ou acide comprend de l'eau dont 10% maximum, de préférence 5% maximum, de préférence 2% maximum, de préférence 0%, peut être remplacé par des agents épaississants. Tout type d'agent épaississant utilisé couramment par les formulateurs est utilisable. L'épaississant ralentit le déplacement vers le bas des particules de polyamides dispersées sous l'effet de la pesanteur. Il empêche donc la sédimentation des particules. Par exemple, les épaississants d'une phase continue aqueuse, notamment dans le domaine cosmétique, sont des substances d'origine le plus souvent végétale : extraits d'algues (alginates) ou de graines (galactomannes, pectines), mais aussi synthétiques (Carbomère = carbopol).

La présente invention a également pour objet un procédé de fabrication de poudre de polyamide selon l'invention, comprenant les étapes suivantes :
A- Agiter un mélange de polyamide, d'eau, et d'acide de Brönsted à base de phosphore (acide P) comprenant de préférence de l'acide phosphorique (H3PO4), pour former une émulsion, dans les conditions suivantes :
   - ledit polyamide de départ comprend plus de 50% molaire d'extrémités amines sur le nombre total d'extrémités amines et acides du polyamide,
   - le ratio molaire [acide P]/[extrémités amines] étant compris dans la gamme de 0,1 à moins de 5, de préférence entre 0,25 et 5, de préférence compris dans la gamme de 0,5 à 3, de préférence de 2 à 3,
   - la quantité en poids de polyamide par rapport au poids total du mélange représente de 0,5 à 60%, de préférence de 10 à 50%, de préférence de 20 à 50%, ou mieux de 30 à 40%,
   - la température du mélange est supérieure à la température de fusion du polyamide ;
   - la vitesse d'agitation et la durée d'agitation sont suffisantes pour former un mélange homogène stable, c'est-à-dire une émulsion de gouttes de polyamide fondu de D50 compris dans la gamme de 100 nm à 50 µm dispersées dans l'eau, puis
B - Refroidir l'émulsion obtenue à l'étape A sous agitation jusqu'à température ambiante (c'est-à-dire une température pouvant être comprise dans la gamme de 5 à 50°C), de sorte qu'on obtient une dispersion aqueuse de particules de polyamide conformes à l'invention.
   Si toutes les conditions de l'étape A sont respectées, l'agitation entraine l'émulsion du polyamide fondu puis une dispersion stable. Sinon, quelle que soit l'agitation, on n'obtient pas d'émulsion en se plaçant en dehors de ces conditions.
   Lors de l'étape A, le ratio molaire [acide P]/[extrémités amines] est généralement compris dans la gamme de 0,1 à moins de 5. Toutefois, on notera les préférences ci-dessous :
   - le ratio molaire [acide P]/[extrémités amines] est compris de préférence entre 0,25 et 5 en particulier dans le cas où ledit polyamide contient moins de 100 µEq/g d'extrémités amines, comme c'est le cas dans les essais 2 à 5 de l'exemple 1 de la présente invention ; et
   - le ratio molaire [acide P]/[extrémités amines] est de préférence inférieur à ces valeurs, et peut être compris dans la gamme plus large de 0,1 (inclus) à moins de 5 (5 exclu), dans le cas où ledit polyamide contient au moins 100 µEq d'extrémités amines par gramme de polyamide comme c'est le cas dans les essais de l'exemple 2 de la présente invention.

   La forme du polyamide de départ n'a aucune importance à l'étape A puisqu'il est fondu pendant le mélange. Par conséquent, toute forme est envisageable pour le polyamide de départ. Pour diminuer le temps de fusion et de mélange, le polyamide de départ est de préférence sous forme de matière divisée, quelle que soit sa taille, généralement comprise entre 60 µm et quelques millimètres, notamment sous la forme de granulé, poudre, fibre, ou autre.
   De préférence, le polyamide est incorporé selon le procédé de l'invention, sous agitation comprise dans la gamme de 100 à 5 000 tours par minute, de préférence de 1000 tr/min à 2 000 tr/min, de préférence de 500 tr/min à 1500 tr/min. On utilise par exemple un agitateur ou une turbine à pâle, à hélice ou à disque, par exemple de marque Rushton® ou équivalent. De préférence la vitesse d'agitation est comprise dans la gamme de 100 à 5000 tr/min et la durée d'agitation comprise dans la gamme de 5 minutes à 1 heure, de préférence dans la gamme de 10 à 30 minutes. Avantageusement, le procédé de l'invention comprend en outre une étape :
C - Récupérer les particules de polyamide, notamment par séparation, filtration et/ou séchage, évaporation, spray drying, de la dispersion aqueuse obtenue à l'étape B.
   Avantageusement, le procédé de l'invention comprend en outre une étape :
D - Re-disperser les particules de PA dans un milieu aqueux neutre ou acide, de sorte qu'on obtient une dispersion comprenant de 0,5 à 90%, de préférence de 0,5 à 70%, en poids de particules de PA.

La présente invention a également pour objet l'utilisation d'une poudre ou d'une composition selon l'invention dans toutes les applications qui exigent à la fois des propriétés de répartition granulométrique étroite dans le cas des poudres, des propriétés de dispersions fines et homogènes, des propriétés filmogènes, adhésives, de compatibilité et d'affinité avec des matrices, en particulier les matrices polaires. Parmi les applications des poudres et dispersions selon l'invention, on peut citer par exemple le can-coating, le papier transfert, le collage, l'entoilage textile, le revêtement de surface, de fils, de fibres, de filaments, de bobines, la fabrication de films, le collage fin, les encres et les peintures, l'ensimage, le traitement des textiles, l'entoilage textile, le traitement du papier, les lubrifiants, les adhésifs hot melt (HMA).

La présente invention a encore pour objet l'utilisation d'une poudre de polyamide selon l'invention pour la fabrication d'un produit cosmétique, pharmaceutique ou de parfumerie, ledit polyamide étant incorporé directement dans la formulation sous la forme d'une poudre ou d'une composition conforme à l'invention.

La présente invention a notamment pour objet une composition selon l'invention telle que définie précédemment, ladite composition étant un produit coloré, non coloré et /ou transparent choisi parmi les produits suivants :
- produits de maquillage pour le visage et le corps humain, tels que fond de teint, crème teintée, poudre libre ou compacte, fard à paupières, mascara, eye liner, rouge à lèvre, vernis à ongles ;
- produits de soins pour le visage et le corps humain, tels que crème, lait, lotion, masque, produit de gommage, produits nettoyants et/ou démaquillants, déodorants, antitranspirants, antiperspirants, produits de rasage, produits d'épilation ;
- produits capillaires, tels que shampooings, produits pour la mise en forme des cheveux, produits de maintien de la coiffure, produits antipelliculaires, produits antichute, produits contre la sécheresse des cheveux, teintures capillaires, produits de décoloration ;
- produits de parfumerie, tels que parfum, lait, crème, poudre libre ou compacte parfumée.

### Exemples

Les exemples ci-dessous illustrent la présente invention sans en limiter la portée. Sauf indication contraire, tous les pourcentages sont en poids.
COPA utilisés :
COPA1 : copolyamide 6/6.6/12, composé de caprolactame, d'hexaméthylènediamine, d'acide adipique, de lauryllactame, de DiéthylèneTriamine, d'acide undécylénique, de Tf= 125°C, contenant 25µeq/g de fins de chaine acide, 63µeq/g de fins de chaine amine et 120µeq/g de fins de chaine alkyle.
COPA2 : copolyamide 6/6.6/12, composé de caprolactame, d'hexaméthylènediamine, d'acide adipique, de lauryllactame, Tf = 125°C contenant 23µeq/g de fins de chaine acide et 319µeq/g de fins de chaine amine.

### Exemple 1

Six essais (Essais 1 à 6) de dispersions aqueuses de COPA1 de ratio molaire H3PO4/fins de chaîne NH2 = 0.25 / 0.5/ 1 / 2 / 3 et 5 ont été réalisés.

Des granulés de COPA1, de l'eau déionisée (de manière à avoir un extrait sec de 30%) et de l'acide phosphorique sont placés dans un autoclave muni d'une cuve en verre d'IL, équipé d'un agitateur type double hélice à 6 pales inclinées. La cuve a un diamètre intérieur de 100 mm et est chauffée par circulation d'huile dans une double enveloppe. L'agitateur a un diamètre de 50 mm. Le milieu est inerté à l'azote et mis en chauffe jusqu'à 150°C matière sous une agitation de 1000 tours par minute (rpm). Cette température est maintenue 30 min puis le milieu est soumis à refroidissement à 50°C. Une dispersion aqueuse de fines particules de polyamide est ainsi obtenue dans les essais 2 à 5, et la distribution de taille des particules est mesurée par diffraction laser.

Résumé des conditions opératoires : Phase de chauffe sous 1000 tr/min, palier 30 min, 150 °C, 1000 tr/min, puis refroidissement.

**Tableau 1**

| N° Essai | Ratio molaire H3PO4/Fins de chaine amine | D50 (µm) | Commentaires |
|---|---|---|---|
| 1 comparatif | 0.25 | | Dispersion non stable / aggrégation |
| 2 invention | 0.5 | 19.3 | Dispersion stable |
| 3 invention | 1 | 1.1 | Dispersion stable |
| 4 invention | 2 | 2.1 | Dispersion stable |
| 5 invention | 3 | 2.1 | Dispersion stable |
| 6 comparatif | 5 | | Formation de mousse/ Dispersion non stable |

Le tableau 1 montre que les essais 2 à 5 selon l'invention permettent d'obtenir une dispersion aqueuse stable de polyamide, lorsque la proportion d'acide phosphorique est conforme à l'invention. Dans l'essai 1, la teneur en H3PO4 est trop faible pour stabiliser des particules et former une dispersion, alors que dans l'essai 6, la teneur est trop importante et un fort moussage est observé, sans possibilité d'obtenir des particules ni une dispersion stable.

Les dispersions obtenues avec un extrait sec de 30% dans les essais 2 à 5 peuvent ensuite être filtrées puis redispersées dans l'eau à l'aide d'un mélangeur. Il est possible d'obtenir des extraits secs supérieurs à 30%, et de formuler facilement en utilisant ces dispersions.

### Augmentation de l'extrait sec : Essai 3.2 selon l'invention :

Dans ces essais, on augmente l'extrait sec des dispersions obtenues.

Pour cela, on réalise dans un premier temps une filtration sur fritté n°4 de l'Essai 3 (ratio molaire = 1) afin d'obtenir une poudre humide. Cette poudre est ensuite redispersée dans l'eau avec un mélangeur, il est ainsi possible d'augmenter l'extrait sec et d'obtenir une texture de pâte visqueuse homogène. On observe ensuite au granulomètre laser que cette manipulation n'influe pas sur la granulométrie des particules.

### Conclusion des essais de l'exemple 1 :

Les dispersions réalisées avec des ratios molaires de 0,5 ; 1 ; 2 et 3 sont préférées car elles présentent des propriétés intéressantes, à savoir des particules de l'ordre du micron, de D50 compris dans la gamme de 1 à 15 µm. De plus, les dispersions de ratio molaire H₃PO₄/NH₂ = 2 et 3 respectivement présentent un état particulièrement homogène stable, et elles peuvent être facilement redispersées et/ou concentrées.
Observations au granulomètre laser : Les solutions de ratio molaire H3PO4/NH2 = 0.5 / 1 / 2 et 3 ont été analysés au granulomètre laser. Deux échantillons ont été analysés pour chaque ratio. Les résultats de distribution granulométrique obtenus (en volume) sont dans le tableau 2 suivant :

**Tableau 2**

| ***Essai*** | ***Essai 2*** | ***Essai 3*** | ***Essai 4*** | ***Essai 5*** |
|---|---|---|---|---|
| ***D50 (µm)*** | *19.370* | *1.129* | *2.119* | 2.126 |
| *% inférieur* | *Taille µm* | *Taille µm* | *Taille µm* | *Taille µm* |
| *10* | *4.2145* | *0.278* | *0.308* | *1.2965* |
| *25* | *10.92* | *0.514* | *1.411* | *1.668* |
| *50* | *19.370* | *1.129* | *2.119* | *2.126* |
| *75* | *25.700* | *1.731* | *3.149* | *2.865* |
| *90* | *31.295* | *2.064* | *4.127* | *3.763* |

### Exemple 2 : Essais 7 et 8 selon l'invention

Le COPA2 est dispersé selon le même procédé que celui de l'exemple 1.
Ratio H3PO4 / fins de chaîne NH2 = 0,1
Vitesse d'agitation : Pour l'essai 7, elle est de 1000 tr/min. Pour l'essai 8 elle est de 1300 tr/min.

La dispersion obtenue est blanche et opaque dans les 2 essais, de pH 6-7.
Dans l'essai 7, l'extrait sec final est 31,54%, et le D50 = 5,39 µm.
Dans l'essai 8, l'extrait sec final est 32,60%, et le D50 = 2,95 µm.

Ces essais de l'exemple 2 illustrent le cas où le ratio H3PO4/extrémités amines peut être inférieur ou égal à 0,25, et se trouver dans la gamme plus large de 0,1 à 5 lorsque ledit polyamide contient au moins 100 µEq/g d'extrémités amines.

On remarque que plus le pourcentage molaire d'extrémités amines sur le nombre total d'extrémités amines et acides du polyamide augmente, plus la quantité d'acide phosphorique nécessaire pour neutraliser et disperser le polyamide est faible. L'acide phosphorique neutralise en même temps plusieurs extrémités NH2 de différentes particules de polyamide. Par effet stérique, l'acide phosphorique empêche les particules à extrémités NH2 de coaguler entre elles, et maintient les particules dispersées dans l'eau.

### Exemple 3 : essais 9 à 11 comparatifs

On réalise 3 essais selon le même procédé et mêmes conditions opératoires que dans l'exemple 1, sauf que l'acide phosphorique est remplacé par un « acide comparatif » différent d'un acide de Brönsted contenant du phosphore. L'acide comparatif est respectivement l'acide heptanoïque dans l'essai 9, l'acide méthane sulfonique dans l'essai 10, et l'acide sulfurique dans l'essai 11.

Dans ces essais 9 à 11, le ratio [acide comparatif] / [fins de chaîne NH2] = 1.

A l'issue de ce procédé, on obtient dans chaque essai 9 à 11, un bloc de polyamide et non pas une dispersion de particules fines de polyamide.

Exemple 4 : Utilisation d'une poudre de Polyamide 10.10 de D50 égal à 20 µm, obtenu selon le procédé décrit dans le brevet, pour la fabrication de composites. Réalisation de résines composites à base d'epoxy : TGMDA / DDS / DDA : systèmes à haute Tg.
- Epoxy : TGMDA : N,N,N', N-tetraglycidyl 4,4'-diaminodiphenylmethane (Araldite MY 720, Huntsman)
- Agent réticulant : DDS/DDA : 4,4' diaminodiphenylsulfone (HT 976, Huntsman) / dicyandiamide
- Catalyseur : 3-3,4-dichlorophenyl)-1,1-dimethylurea (diuron)

Composition des résines : 1 groupe epoxy pour 0.85 hydrogène d'amine des DDS/DDA ; Rapport DDA/DDS : 0.25 ; Rapport diuron / DDS : 0.01. On teste 3 teneurs différentes en poudre PA10.10 dans la résine : 0 phr, 10 phr, 20 phr, phr signifiant « partie pour cent de résine, en poids ». Cycle de réticulation : 1h à 110°C, 1h à 120°C, 1h à 130°C, 1h à 140°C, 1h à 150°C, 1h à 180°C

Le facteur d'intensité de contrainte critique K1C est mesuré (norme ASTMD5045, en MPa/√m, il définit pour le matériau la valeur critique de contrainte au-delà de laquelle se produit la rupture) :

| Teneur en poudre (phr) | K1C |
|---|---|
| 0 | 0.65 |
| 10 | 0.7 |
| 20 | 0.8 |

L'utilisation de poudre selon l'invention améliore et confère une excellente ténacité à la rupture de la résine epoxy.

## Revendications

1. Poudre à base de particules de polyamide dans laquelle :
- ledit polyamide comporte plus de 50% molaire d'extrémités amines sur le nombre total d'extrémités amines et acides du polyamide;
- lesdites particules comportent à leur surface des groupements amines primaires au moins partiellement neutralisés par un acide de Brönsted à base de phosphore, de préférence l'acide phosphorique ;
- la D50 des particules est comprise dans la gamme de 100 nm à 50 µm, de préférence de 100 nm à 20 µm,
la D50 correspondant à la taille moyenne en volume, mesurée selon la norme ISO 9276 - parties 1 à 6.

2. Poudre selon la revendication 1, dans laquelle le polyamide comprend au moins un des monomères suivants : 4.6, 4.T, 5.6, 5.9, 5.10, 5.12, 5.13, 5.14, 5.16, 5.18, 5.36, 6, 6.6, 6.9, 6.10, 6.12, 6.13, 6.14, 6.16, 6.18, 6.36, 6 .T, 9, 10.6, 10.9, 10.10, 10.12, 10.13, 10.14, 10.16, 10.18, 10.36, 10.T, 11, 12, 12.6, 12.9, 12.10, 12.12, 12.13, 12.14, 12.16, 12.18, 12.36, 12.T, et leurs mélanges ; et de préférence est choisi parmi le PA 11, le PA 12, le PA 10.10, le PA 6, le PA 6.10, PA 6.12, PA 10.12, PA 6.14 et/ou PA 6.6/6, le PA 11/10.10, et leurs mélanges.

3. Poudre selon la revendication 1 ou 2, dans laquelle le polyamide comprend au moins un copolyamide, de préférence choisi parmi : PA 6/6.6/12, PA 6/6.6/11/12, PA 6/12, PA 6.9/12, PA Pip.9/Pip.12/11, PA 6/IPD.6/12, PA IPD.9/12, PA6/MPMD.12/12, PA 6/6.12/12, PA 6/Pip.12/12, PA 6/6.6/6.10/6.1, PA 6.10/Pip.10/Pip.12, PA 6/11/12, PA Pip.12/12, PA IPD.10/12, PA Pip.10/12, PA 6/11, PA Pip.10/11/Pip.9, PA 6/6.6/6.10, et leurs mélanges.

4. Poudre selon l'une quelconque des revendications 1 à 3, **caractérisée en ce qu'**elle contient des particules de forme sphéroïdale et de surface poreuse, de préférence creuses.

5. Poudre selon l'une quelconque des revendications 1 à 3, **caractérisée en ce que** le coeur des particules contient au moins un produit cosmétique, pharmaceutique ou de parfumerie.

6. Composition fluide homogène comprenant :
- de 0,5 à 90% en poids de poudre selon l'une quelconque des revendications 1 à 4,
- de 10 à 99,5% en poids d'un milieu aqueux de pH neutre ou acide.

7. Composition selon la revendication 6, **caractérisée en ce qu'**elle comprend :
- de 0,5 à 60% en poids de poudre selon l'une quelconque des revendications 1 à 4, et
- de 40 à 99,5% en poids d'un milieu aqueux de pH neutre ou acide, de préférence de pH compris dans la gamme de 1 à 7, de préférence de 4 à 6,5, de préférence de l'eau, ladite composition formant une dispersion aqueuse stable sans tensioactif.

8. Composition selon l'une quelconque des revendications 6 ou 7, dans laquelle ledit milieu aqueux de pH neutre ou acide comprend de l'eau dont 10% maximum, de préférence 5% maximum, de préférence 2% maximum, de préférence 0%, peut être remplacé par des agents épaississants.

9. Composition selon l'une quelconque des revendications 6 à 8, ladite composition étant un produit coloré, non coloré et /ou transparent choisi parmi les produits suivants :
- produits de maquillage pour le visage et le corps humain, tels que fond de teint, crème teintée, poudre libre ou compacte, fard à paupières, mascara, eye liner, rouge à lèvre, vernis à ongles ;
- produits de soins pour le visage et le corps humain, tels que crème, lait, lotion, masque, produit de gommage, produits nettoyants et/ou démaquillants, déodorants, antitranspirants, antiperspirants, produits de rasage, produits d'épilation ;
- produits capillaires, tels que shampooings, produits pour la mise en forme des cheveux, produits de maintien de la coiffure, produits antipelliculaires, produits antichute, produits contre la sécheresse des cheveux, teintures capillaires, produits de décoloration ;
- produits de parfumerie, tels que parfum, lait, crème, poudre libre ou compacte parfumée.

10. Procédé de fabrication de poudre de polyamide selon l'une quelconque des revendications 1 à 4, comprenant les étapes suivantes :
A- Agiter un mélange de polyamide, d'eau, et d'acide de Brönsted à base de phosphore (acide P), comprenant de préférence de l'acide phosphorique (H3PO4), pour former une émulsion, dans les conditions suivantes :
- ledit polyamide de départ comprend plus de 50% molaire d'extrémités amines sur le nombre total d'extrémités amines et acides du polyamide,
- le ratio molaire [acide P]/[extrémités amines] est compris dans la gamme de 0,1 à 5 ;
- la quantité en poids de polyamide par rapport au poids total du mélange représente de 0,5 à 60%,
- la température du mélange est supérieure à la température de fusion du polyamide ;
- la vitesse d'agitation et la durée d'agitation sont suffisantes, de préférence la vitesse d'agitation est comprise dans la gamme de 100 à 5000 tr/min et la durée d'agitation comprise dans la gamme de 5 minutes à 1 heure, pour former un mélange homogène stable ;
B - Refroidir l'émulsion obtenue à l'étape A sous agitation, de préférence jusqu'à température ambiante, de sorte que l'on obtient une dispersion aqueuse de particules de polyamide conformes à l'une quelconque des revendications 1 à 4.

11. Procédé selon la revendication 10, dans lequel, au cours de l'étape A :
- le ratio molaire [acide P]/[extrémités amines] est compris entre 0,25 et 5 dans le cas où ledit polyamide contient moins de 100 µEq/g d'extrémités aminés ; et
- le ratio molaire [acide P]/[extrémités amines] est compris dans la gamme de 0,1 à moins de 5, dans le cas où ledit polyamide contient au moins 100 µEq/g d'extrémités amines.

12. Procédé selon l'une quelconque des revendications 10 ou 11, comprenant en outre une étape :
C - Récupérer les particules de polyamide, notamment par séparation, filtration et/ou séchage, évaporation, spray drying, de la dispersion aqueuse obtenue à l'étape B.

13. Procédé selon l'une quelconque des revendications 10 à 12, comprenant en outre une étape :
D - Re-disperser les particules de PA dans un milieu aqueux neutre ou acide, de sorte que l'on obtient une dispersion comprenant de 0,5 à 90%, en poids de particules de PA.

14. Utilisation de poudre selon l'une quelconque des revendications 1 à 4 ou d'une composition selon l'une quelconque des revendications 6 à 8, dans le can-coating, le papier transfert, le collage, l'entoilage textile, le revêtement de surface, de fils, de fibres, de filaments, de bobines, la fabrication de films, le collage fin, les encres, les peintures, l'ensimage, le traitement des textiles, le traitement du papier, les lubrifiants, les adhésifs hot melt (HMA), les composites.

15. Utilisation de poudre selon l'une quelconque des revendications 1 à 4 ou d'une composition selon l'une quelconque des revendications 6 à 8, pour la fabrication d'un produit cosmétique, pharmaceutique ou de parfumerie.

## Patentansprüche

1. Pulver auf Basis von Polyamidteilchen, wobei:
- das Polyamid mehr als 50 Mol-% Amin-Endgruppen, bezogen auf die Gesamtzahl von Amin- und Säure-Endgruppen des Polyamids, umfasst;
- die Teilchen auf ihrer Oberfläche zumindest teilweise durch eine Brönsted-Säure auf Phosphorbasis, vorzugsweise Phosphorsäure, neutralisierte primäre Amingruppen tragen;
- der D50-Wert der Teilchen im Bereich von 100 nm bis 50 µm, vorzugsweise von 100 nm bis 20 µm, liegt,
wobei der D50-Wert der gemäß der ISO-Norm 9276, Teile 1 bis 6, gemessenen volumenmittleren Teilchengröße entspricht.

2. Pulver nach Anspruch 1, wobei das Polyamid mindestens eines der folgenden Monomere: 4.6, 4.T, 5.6, 5.9, 5.10, 5.12, 5.13, 5.14, 5.16, 5.18, 5.36, 6, 6.6, 6.9, 6.10, 6.12, 6.13, 6.14, 6.16, 6.18, 6.36, 6.T, 9, 10.6, 10.9, 10.10, 10.12, 10.13, 10.14, 10.16, 10.18, 10.36, 10.T, 11, 12, 12.6, 12.9, 12.10, 12.12, 12.13, 12.14, 12.16, 12.18, 12.36, 12.T und Mischungen davon umfasst und vorzugsweise aus PA 11, PA 12, PA 10.10, PA 6, PA 6.10, PA 6.12, PA 10.12, PA 6.14 und/oder PA 6.6/6, PA 11/10.10 und Mischungen davon ausgewählt ist.

3. Pulver nach Anspruch 1 oder 2, wobei das Polyamid mindestens ein Copolyamid umfasst, das vorzugsweise aus PA 6/6.6/12, PA 6/6.6/11/12, PA 6/12, PA 6.9/12, PA Pip.9/Pip.12/11, PA 6/IPD.6/12, PA IPD.9/12, PA6/MPMD.12/12, PA 6/6.12/12, PA 6/Pip.12/12, PA 6/6.6/6.10/6.I, PA 6.10/Pip.10/Pip.12, PA 6/11/12, PA Pip.12/12, PA IPD.10/12, PA Pip.10/12, PA 6/11, PA Pip.10/11/Pip.9, PA 6/6.6/6.10 und Mischungen davon ausgewählt ist.

4. Pulver nach einem der Ansprüche 1 bis 3, **dadurch gekennzeichnet, dass** es kugelförmige Teilchen mit einer porösen Oberfläche, die vorzugsweise hohl sind, enthält.

5. Pulver nach einem der Ansprüche 1 bis 3, **dadurch gekennzeichnet, dass** der Kern der Teilchen mindestens ein kosmetisches Produkt, pharmazeutisches Produkt oder Parfümerieprodukt enthält.

6. Homogene fließfähige Zusammensetzung, umfassend:
- 0,5 bis 90 Gew.-% Pulver nach einem der Ansprüche 1 bis 4,
- 10 bis 99,5 Gew.-% eines wässrigen Mediums mit neutralem oder saurem pH-Wert.

7. Zusammensetzung nach Anspruch 6, **dadurch gekennzeichnet, dass** sie Folgendes umfasst:
- 0,5 bis 60 Gew.-% Pulver nach einem der Ansprüche 1 bis 4 und
- 40 bis 99,5 Gew.-% eines wässrigen Mediums mit neutralem oder saurem pH-Wert, vorzugsweise mit einem pH-Wert im Bereich von 1 bis 7, vorzugsweise 4 bis 6,5, vorzugsweise Wasser, wobei die Zusammensetzung ohne Tensid eine stabile wässrige Dispersion bildet.

8. Zusammensetzung nach einem der Ansprüche 6 oder 7, wobei das wässrige Medium mit neutralem oder saurem pH-Wert Wasser umfasst, wovon maximal 10 %, vorzugsweise maximal 5 %, vorzugsweise maximal 2 %, vorzugsweise 0 %, durch Verdickungsmittel ersetzt sein können.

9. Zusammensetzung nach einem der Ansprüche 6 bis 8, wobei es sich bei der Zusammensetzung um ein gefärbtes, ungefärbtes und/oder transparentes Produkt handelt, das aus den folgenden Produkten ausgewählt ist:
- Make-up-Produkten für Gesicht und Körper des Menschen, wie Grundierung, getönter Creme, losem oder kompaktem Puder, Lidschatten, Mascara, Eyeliner, Lippenstift und Nagellack;
- Pflegeprodukten für Gesicht und Körper des Menschen, wie Creme, Milch, Lotion, Maske, Peeling-Produkten, Reinigungs- und/oder Abschminkprodukten, Deodorantien, Antitranspirantien, Antiperspirantien, Rasierprodukten und Haarentfernungsprodukten;
- Haarprodukten, wie Shampoos, Produkten für das Formen der Haare, Produkten zur Erhaltung der Frisur, Produkten gegen Schuppen, Produkten gegen Haarausfall, Produkten gegen Haartrockenheit, Haarfärbemitteln und Entfärbungsprodukten;
- Parfümerieprodukten, wie Parfüm, Milch, Creme und parfümiertem losem oder kompaktem Puder.

10. Verfahren zur Herstellung von Polyamidpulver nach einem der Ansprüche 1 bis 4, das die folgenden Schritte umfasst:
A - Rühren einer Mischung von Polyamid, Wasser und Brönsted-Säure auf Phosphorbasis (P-Säure), die vorzugsweise Phosphorsäure (H3PO4) umfasst, zur Bildung einer Emulsion unter den folgenden Bedingungen:
- das Ausgangspolyamid umfasst mehr als 50 Mol-% Amin-Endgruppen, bezogen auf die Gesamtzahl von Amin- und Säure-Endgruppen des Polyamids;
- das Molverhältnis [P-Säure]/[Amin-Endgruppen] liegt im Bereich von 0,1 bis 5;
- die Gewichtsmenge an Polyamid, bezogen auf das Gesamtgewicht der Mischung, macht 0,5 bis 60 % aus,
- die Temperatur der Mischung liegt über dem Schmelzpunkt des Polyamids;
- die Rührgeschwindigkeit und die Rührdauer reichen zur Bildung einer stabilen homogenen Mischung aus, vorzugsweise liegt die Rührgeschwindigkeit im Bereich von 100 bis 5000 U/min und die Rührdauer im Bereich von 5 Minuten bis 1 Stunde;
B - Abkühlen der in Schritt A erhaltenen Emulsion unter Rühren, vorzugweise auf Umgebungstemperatur, so dass man eine wässrige Dispersion von Polyamidteilchen nach einem der Ansprüche 1 bis 4 erhält.

11. Verfahren nach Anspruch 10, bei dem in Schritt A:
- das Molverhältnis [P-Säure]/[Amin-Endgruppen] zwischen 0,25 und 5 liegt, wenn das Polyamid weniger als 100 µÄq./g Amin-Endgruppen enthält; und
- das Molverhältnis [P-Säure]/[Amin-Endgruppen] im Bereich von 0,1 bis weniger als 5 liegt, wenn das Polyamid mindestens 100 µÄq./g Amin-Endgruppen enthält.

12. Verfahren nach einem der Ansprüche 10 oder 11, das außerdem folgenden Schritt umfasst:
C - Gewinnen der Polyamidteilchen, insbesondere durch Abtrennen, Filtrieren und/oder Trocknen, Eindampfen, Sprühtrocknen, aus der in Schritt B erhaltenen wässrigen Dispersion.

13. Verfahren nach einem der Ansprüche 10 bis 12, das außerdem folgenden Schritt umfasst:
D - Redispergieren der PA-Teilchen in einem neutralen oder sauren wässrigen Medium, so dass man eine Dispersion, die 0,5 bis 90 Gew.-% PA-Teilchen umfasst, erhält.

14. Verwendung von Pulver nach einem der Ansprüche 1 bis 4 oder einer Zusammensetzung nach einem der Ansprüche 6 bis 8 bei der Dosenbeschichtung, in Transferpapier, beim Kleben, bei der Textilbespannung, bei der Beschichtung von Oberflächen, Drähten, Fasern, Filamenten und Spulen, bei der Herstellung von Folien, beim Feinkleben, in Tinten, in Lacken, beim Schmälzen, bei der Behandlung von Textilien, bei der Behandlung von Papier, in Schmiermitteln, in Schmelzklebstoffen (HMA) und in Verbundwerkstoffen.

15. Verwendung von Pulver nach einem der Ansprüche 1 bis 4 oder einer Zusammensetzung nach einem der Ansprüche 6 bis 8 zur Herstellung eines kosmetischen Produkts, pharmazeutischen Produkts oder Parfümerieprodukts.

## Claims

1. Powder based on polyamide particles, in which:
- said polyamide comprises more than 50 mol% of amine ends among the total number of amine and acid ends of the polyamide;
- said particles bear, on their surface, primary amine groups at least partially neutralized by a phosphorus-based Brønsted acid, preferably phosphoric acid;
- D50 of the particles is in the range from 100 nm to 50 µm, preferably from 100 nm to 20 µm,
D50 corresponding to the average size by volume, measured according to standard ISO 9276 - parts 1 to 6.

2. Powder according to Claim 1, in which the polyamide comprises at least one of the following monomers: 4.6, 4.T, 5.6, 5.9, 5.10, 5.12, 5.13, 5.14, 5.16, 5.18, 5.36, 6, 6.6, 6.9, 6.10, 6.12, 6.13, 6.14, 6.16, 6.18, 6.36, 6.T, 9, 10.6, 10.9, 10.10, 10.12, 10.13, 10.14, 10.16, 10.18, 10.36, 10.T, 11, 12, 12.6, 12.9, 12.10, 12.12, 12.13, 12.14, 12.16, 12.18, 12.36, 12.T, and mixtures thereof; and is preferably selected from PA 11, PA 12, PA 10.10, PA 6, PA 6.10, PA 6.12, PA 10.12, PA 6.14 and/or PA 6.6/6, PA 11/10.10, and mixtures thereof.

3. Powder according to Claim 1 or 2, in which the polyamide comprises at least one copolyamide, preferably selected from: PA 6/6.6/12, PA 6/6.6/11/12, PA 6/12, PA 6.9/12, PA Pip.9/Pip.12/11, PA 6/IPD.6/12, PA IPD.9/12, PA6/MPMD.12/12, PA 6/6.12/12, PA 6/Pip.12/12, PA 6/6.6/6.10/6.I, PA 6.10/Pip.10/Pip.12, PA 6/11/12, PA Pip.12/12, PA IPD.10/12, PA Pip.10/12, PA 6/11, PA Pip.10/11/Pip.9, PA 6/6.6/6.10, and mixtures thereof.

4. Powder according to any one of Claims 1 to 3, **characterized in that** it contains particles of spheroidal shape and with a porous surface, preferably hollow.

5. Powder according to any one of Claims 1 to 3, **characterized in that** the core of the particles contains at least one cosmetic, pharmaceutical or perfumery product.

6. Homogeneous fluid composition comprising:
- from 0.5 to 90 wt% of powder according to any one of claims 1 to 5,
- from 10 to 99.5 wt% of an aqueous medium with neutral or acid pH.

7. Composition according to Claim 6, **characterized in that** it comprises:
- from 0.5 to 60 wt% of powder according to any one of Claims 1 to 4, and
- from 40 to 99.5 wt% of an aqueous medium with neutral or acid pH, preferably with pH in the range from 1 to 7, preferably from 4 to 6.5, preferably water, said composition forming a stable aqueous dispersion without surfactant.

8. Composition according to either of Claims 6 and 7, in which said aqueous medium with neutral or acid pH comprises water, of which at most 10%, preferably at most 5%, preferably at most 2%, preferably 0%, may be replaced with thickeners.

9. Composition according to any one of Claims 6 to 8, said composition being a colored, noncolored and/or transparent product selected from the following products:
- makeup products for the human face and body, such as foundation, tinted cream, loose or compacted powder, eye shadow, mascara, eye liner, lipstick, nail varnish;
- care products for the human face and body, such as cream, milk, lotion, mask, peeling product, cleansing and/or makeup removal products, deodorants, antiperspirants, shaving products, hair removal products;
- hair products, such as shampoos, hair shaping products, styling products, antidandruff products, products against hair loss, products against dryness of the hair, hair coloring products, bleaching products;
- perfumery products, such as perfume, milk, cream, loose or compacted perfumed powder.

10. Method of manufacturing polyamide powder according to any one of Claims 1 to 4, comprising the following steps:
A- Stir a mixture of polyamide, water, and phosphorus-based Brønsted acid (acid P), preferably comprising phosphoric acid (H₃PO₄), to form an emulsion, in the following conditions:
- said starting polyamide comprises more than 50 mol% of amine ends among the total number of amine and acid ends of the polyamide,
- the molar ratio [acid P]/[amine ends] is in the range from 0.1 to 5;
- the amount of polyamide by weight relative to the total weight of the mixture represents from 0.5 to 60%,
- the temperature of the mixture is above the melting point of the polyamide;
- the stirring speed and stirring time are sufficient, preferably the stirring speed is in the range from 100 to 5000 rev/min and the stirring time is in the range from 5 minutes to 1 hour, to form a stable homogeneous mixture;
B - Cool the emulsion obtained in step A with stirring, preferably to room temperature, so that an aqueous dispersion of polyamide particles according to any one of Claims 1 to 4 is obtained.

11. Method according to Claim 10, in which, during step A:
- the molar ratio [acid P]/[amine ends] is between 0.25 and 5 in the case when said polyamide contains less than 100 µEq/g of amine ends; and
- the molar ratio [acid P]/[amine ends] is in the range from 0.1 to less than 5, in the case when said polyamide contains at least 100 µEq/g of amine ends.

12. Method according to either of Claims 10 and 11, further comprising a step:
C - Recover the polyamide particles, notably by separation, filtration and/or drying, evaporation, spray drying, from the aqueous dispersion obtained in step B.

13. Method according to any one of Claims 10 to 12, further comprising a step:
D - Re-disperse the PA particles in a neutral or acid aqueous medium, so that a dispersion is obtained comprising from 0.5 to 90%, by weight of PA particles.

14. Use of powder according to any one of Claims 1 to 4 or of a composition according to any one of Claims 6 to 8, in can coating, release paper, gluing, fabric covering, surface coating, coating of wires, fibers, filaments, coils, manufacture of films, fine gluing, inks, paints, size, textile treatments, treatment of paper, lubricants, hot melt adhesives (HMA), composites.

15. Use of powder according to any one of Claims 1 to 4 or of a composition according to any one of Claims 6 to 8, for manufacturing a cosmetic, pharmaceutical or perfumery product.
